## Europäisches Patentamt

## European Patent Office

## Office européen des brevets

(19)

(11) Veröffentlichungsnummer: **0 192 024**
**B1**

(12) **EUROPÄISCHE PATENTSCHRIFT**

(45) Veröffentlichungstag der Patentschrift:
07.06.89

(21) Anmeldenummer: 86100144.4

(22) Anmeldetag: 08.01.86

(51) Int. Cl.⁴: **A 61 B 19/00**, A 61 M 5/00,
H 05 B 6/80

(54) Zum Erwärmen von im Beutel bevorratetem Blut oder Blutderivaten dienendes Mikrowellengerät.

(30) Priorität: 23.01.85 DE 3502095
07.12.85 DE 3543308

(43) Veröffentlichungstag der Anmeldung:
27.08.86 Patentblatt 86/35

(45) Bekanntmachung des Hinweises auf die Patenterteilung: 07.06.89 Patentblatt 89/23

(84) Benannte Vertragsstaaten:
AT BE CH FR GB IT LI LU NL SE

(56) Entgegenhaltungen:
EP--B-- 0 012 123
CH--A-- 444 338
DE--A-- 2 320 440
DE--A-- 2 418 155
US--A-- 3 518 393

(73) Patentinhaber: Zeipel, Kurt
Spandauer Strasse 9
D-3406 Bovenden-Lenglern (DE)

(72) Erfinder: Zeipel, Kurt
Spandauer Strasse 9
D-3406 Bovenden-Lenglern (DE)

(74) Vertreter: Patentanwälte Dipl.-Ing. Rudolf Bibrach
Dipl.-Ing. Elmar Rehberg
Postfach 1453 Pütterweg 6
D-3400 Göttingen (DE)

EP 0 192 024 B1

## Beschreibung

Die Erfindung bezieht sich auf ein Mikrowellengerät zum Erwärmen von in einem Beutel bevorratetem Blut oder Blutderivaten mit den Merkmalen des Oberbegriffs des Anspruches 1.

Blutkonserven werden bekanntlich auch in Kunststoffbeuteln bevorratet und bei ca. 4-5 °C gelagert. Blutübertragungen können jedoch nur mit vorgewärmtem Blut vorgenommen werden, welches der Körpertemperatur angepaßt ist und z. B. auf 27 oder auch 33 °C erwärmt ist.

Aus der US-A-3 518 393 ist ein Mikrowellengerät der eingangs beschriebenen Art bekannt. Zwischen zwei als Elektroden ausgebildete Platten wird ein elektromagnetisches Feld erzeugt. An einer der beiden Elektroden ist ein Haken vorgesehen, an dem der Beutel befestigbar ist. Die andere Elektrode wird mittels eines Federtriebes gegen den Beutel gedrückt, so daß dieser zwischen den beiden Elektroden eingespannt ist. Die beiden Elektroden sind drehbar gelagert und werden von einem reversierend arbeitenden Antrieb angesteuert. Nachteilig ist, daß nur eine vergleichsweise geringe Durchmischung des sich im Beutel befindlichen Blutes bzw. Blutderivates stattfindet. Es besteht somit die Gefahr, daß örtliche Überhitzungen auftreten und somit das sich im Beutel befindliche Blut unbrauchbar wird.

Durch die DE-AS 23 20 440 und die DE-OS 24 18 155 sind derartige Mikrowellengeräte zur Erwärmung von Blut bekannt, bei denen das Blut in Flaschen oder auch in Beuteln dem Erwärmungsvorgang unterzogen wird. Die Haltevorrichtung für den Behälter des Bluts besteht aus zwei gegeneinander verspannbare Backen in Halbschalenform, so daß auch ein flacher Beutel durch diese Halbschalen mehr oder weniger in eine zylindrische Form gepreßt den Mikrowellen ausgesetzt wird. Damit entstehen unterschiedliche Schichtdicken in der Folge einer ungleichen Erwärmung des Bluts, die erst recht durch eine Durchmischung des Bluts ausgeglichen werden muß. Da die Achse der Haltevorrichtung jedoch quer, d. h. senkrecht zu der Drehachse des Antriebsmotors angeordnet ist, findet nur eine vergleichsweise bescheidene Durchmischung des Flüssigkeitsinhalts statt. Derartige Flaschen oder zylinderförmige Behälter weisen üblicherweise keine Stutzten oder schlauchförmige Verlängerungen auf, so daß das Problem der Erwärmung der Flüssigkeit bei besonders geringen Schichtdicken nicht auftritt.

Die CH-PS 444 338 zeigt ein Mikrowellengerät anderer Bauart, bei der die Drehachse des Antriebs und die Achse des flaschenförmigen Behälters miteinander fluchten. Diese Achsen sind schrägstehend zur Horizontalen angeordnet, so daß bei einer Drehbewegung praktisch nur die Wandung des Behälters gegenüber dem Flüssigkeitsinhalt gedreht wird. Der Motor kann umlaufend oder hin- und hergehend angetrieben werden. Auch eine Taumelbewegung soll möglich sein, ohne daß erkennbar ist, wie diese erreicht werden könnte.

Der Erfindung liegt die Aufgabe zugrunde, eine Vorrichtung der eingangs beschriebenen Art aufzuzeigen, mit der in sehr kurzer Zeit nur die tatsächlich benötigte Blutmenge ohne Beeinträchtigung des Blutes durch ungleiche Erwärmung, insb. örtliche Überhitzung, erwärmt werden kann. Dabei soll die Erwärmung natürlich trotzdem in sehr kurzer Zeit und unter besonders intensiver Durchmischung bzw. Durchwirbelung der Flüssigkeit im Beutel stattfinden.

Erfindungsgemäß wird dies dadurch erreicht, daß die Aufnahmevorrichtung in einem Mikrowellenerwärmungsraum vorgesehen ist und eine Aufspannplatte zur Aufnahme des Beutels aufweist, daß die ebene Fläche der Aufspannplatte schrägstehend zu der Drehachse des als Elektromotors ausgebildeten Antriebs angeordnet und mit einem Ende der Drehachse verbunden ist. Damit ist eine schnelle und intensive Erwärmung und Durchmischung ohne Verwendung eines zusätzlichen Einmal-Verbrauchsmaterials möglich. Für die Aufheizung eines Beutels mit einem Inhalt von 400-500 ml von 4 °C auf 32 °C wird eine Aufheizzeit von ca. 110 sec benötigt. Diese kurze Zeit ist immer ausreichend, um lediglich das unmittelbar benötigte Blut kurzfristig zu erwärmen. Eine Erwärmung auf Vorrat ist damit nicht mehr erforderlich. Wesentlich für die Erfindung ist, es, daß die Achse der Aufnahmevorrichtung nicht nur quer, sondern schrägstehend zu der Drehachse des Elektromotors angeordnet ist, und daß die Aufnahmevorrichtung eine Aufspannplatte, also eine im wesentlichen ebene Fläche, zur Aufnahme des Beutels aufweist. Damit ist es nämlich möglich, den Beutel flachliegend und weitgehend ausgestreckt auf der Aufspannplatte aufzunehmen und in Verbindung mit dem reversierenden Antrieb durch den Elektromotor in eine besondere Schräg-Taumelbewegung zu versetzen, die eine besonders intensive Durchmischung und Durchwirbelung des zu erwärmenden Bluts während des Erwärmungsvorgangs nach sich zieht. Das einzelne Blutpartikelchen wird nicht nur entsprechend seiner unterschiedlichen Entfernung von der Drehachse unterschiedlich beschleunigt bzw. verzögert; die Beschleunigungs- und Verzögerungskräfte sind darüberhinaus in Verbindung mit schrägstehenden Flächen der Beutelwandung zu sehen, so daß die einzelnen Blutpartikelchen an diesen Schrägflächen eine Richtungsänderung ihrer Bewegung erhalten, die eine in Richtung der Drehachse bzw. parallel zu dieser verlaufende Komponente aufweist. Natürlich wirkt außerdem die Schwerkraft auf jedes einzelne Partikelchen ein. Durch den Einfluß dieser drei Komponenten ergibt sich die besondere Schräg-Taumelbewegung in Form einer intensiven Durchmischung, so daß der Inhalt des Beutels nicht nur schnell, sondern über den Beutelquerschnitt auch gleichmäßig erwärmt wird. Da die Vorrichtung nur kurzzeitig im Einsatz ist, ist sie schnell für weitere Blutkonserven verfügbar, so daß auch mehrere Operationsräume mit einer erfindungsgemäßen Vorrichtung bedienbar sind. Es entstehen auch keine Sterilisationsprobleme, da das Blut während der Erwärmung in dem Beutel bleibt, in welchem es auch aufbewahrt wurde.

Die Aufspannplatte kann etwa in einem Winkel von 45° zur Horizontalen schrägstehend angeordnet sein, wobei der Antrieb für die Drehbewegung zweckmäßig einen Elektromotor aufweist, der reversierend gesteuert ist. Durch diese Schub-Dreh-Bewegung des Beutels findet eine ständige Umwälzung und eine homogene Erwärmung des Bluts statt. Ein solcher reversierender Antrieb ist für eine gleichmäßige Durchmischung besonders geeignet.

Der Elektromotor kann in einem Winkel von etwa 350° hin- und herschwenkend gesteuert sein. Dieser Winkelbereich hat sich in Verbindung mit einer im Winkel von 45° schrägstehend angeordneten Aufnahmeplatte als besonders vorteilhaft erwiesen. Weiterhin ist es sinnvoll, wenn die Aufspannplatte aus einem vermittels Mikrowellen nicht oder möglichst wenig sich erwärmenden Material, insbesondere Kunststoff, besteht, damit tatsächlich nur das Blut aufgewärmt wird und nicht die Aufspannplatte.

Somit können auch keine Nachheizerscheinungen zwischen Aufspannplatte und Flut eintreten, da die Aufspannplatte nicht oder möglichst wenig miterwärmt wird.

Die Aufspannplatte ist zweckmäßig mit einem Fixiergurt, einem Spannbügel o. dgl. zur Fixierung des Beutels versehen, damit die Blutkonserve schnell und unproblematisch an der schrägstehenden Aufspannplatte befestigt werden kann.

In der dem Beutel zugekehrten Oberfläche der Aufspannplatte kann ein Thermoelement zur Steuerung des Erzeugers der Mikrowellen vorgesehen sein. Dieses Thermoelement zeigt die Temperatur an, wobei diese Temperatur möglichst nahe an dem Beutel bzw. dem Blut gemessen wird und nicht etwa die Temperatur der Aufspannplatte. Über eine geeignete Steuereinrichtung läßt sich die Aufwärmtemperatur für das Blut je nach den Erfordernissen einstellen, beispielsweise auf 27 °C oder auch auf 33 °C, um einige bevorzugte Aufwärmtemperaturen zu nennen.

Eine mit besonderen Vorteilen ausgestattete, erfindungsgemäße Weiterbildung der Aufnahmevorrichtung ergibt sich dann, wenn die Aufnahmevorrichtung eine Abdeckplatte aufweist, zwischen der und der Aufnahmeplatte der Beutel im Sinne gleicher Schichtdicken flachgepreßt gehalten ist, und wenn die Aufnahmeplatte und die Abdeckplatte über einen Teil ihrer Längs- und Querausdehnung entsprechend der Anordnung von Stutzen am Beutel mit einem die Mikrowellen reflektierenden Material belegt sind. Auch dabei ist die Achse der Aufnahmevorrichtung bzw. der Aufspannplatte schrägstehend zu der Drehachse des Elektromotors angeordnet, also in einem von 90° verschiedenen Winkel. Zu der Aufspannplatte tritt jedoch nun eine Abdeckplatte hinzu, die natürlich ebenfalls als ebene Fläche ausgebildet ist und auch vergleichbare Größenordnung zu der Aufspannplatte aufweist. Zwischen diesen beiden ebenen Platten wird der Beutel in der Aufnahmevorrichtung flachgepreßt gehalten, so daß die Aufwölbungen des nachgiebigen Beutels nicht nur einseitig, sondern jetzt zweiseitig beseitigt sind und außerdem eine durch den Spanngurt ohne Abdeckplatte bewirkte Kammerbildung beseitigt ist. Im Gegenteil, die Schichtdicke des Beutels ist über nahezu die gesamte Beutelfläche konstant, da die Abdeckplatte genau oder zumindest angenähert parallel zu der Aufspannplatte ausgerichtet in der Haltestellung an dem Beutel zur Anlage kommt. Die gleiche Schichtdicke der Flüssigkeit im Beutel garantiert nicht nur eine gleichmäßige Erwärmung über die Fläche. An diesem Querschnitt tritt vielmehr auch keine Verengung mehr auf, so daß die Bewegungsrichtungsveränderung der einzelnen Blutpartikel entsprechend einer in Richtung der Drehachse wirkenden Kraftkomponente sich voll auswirken kann und somit die besondere Schräg-Taumeldurchmischung der Flüssigkeit des Beutels ungehindert entsteht. Von dieser Schräg-Taumeldurchmischung wird der wesentliche Beutelinhalt erfaßt, jedoch nehmen die Teile der Flüssigkeit, die in am Beutel vorgesehenen Stutzen, schlauchförmigen Verlängerungen o. dgl. vorgesehen sind, nicht bzw. nicht voll an dieser Taumel- und Durchmischungsbewegung teil. Da in einem solchen Stutzen jedoch eine im Vergleich zu der übrigen Schichtdicke des Beutels wesentlich reduzierte Schichtdicke vorliegt, wird in diesem durch die Stutzen und schlauchförmigen Verlängerungen am Beutel vorgegebene Fläche sowohl die Aufspannplatte als auch die Abdeckplatte mit reflektierendem Material belegt bzw. versehen, so daß zumindest ein Teil der Mikrowellen durch Reflektion von diesem Bereich des Beutels und der darin befindlichen Flüssigkeit ferngehalten wird. Da sich die Aufspannplatte und die Abdeckplatte auf Abstand voneinander befinden, kann ein Teil der Mikrowellen trotzdem durch den gebildeten Spalt auf die Flüssigkeit in den Stutzen einwirken, so daß hier eine vergleichsweise langsamere Erwärmung stattfindet. Diese langsamere Erwärmung ist durchaus erwünscht, um auf jeden Fall Überhitzungserscheinungen der empfindlichen Flüssigkeit zu vermeiden. Die hervorragende Durchmischung der Flüssigkeit infolge der Schräg-Taumelbewegung wirkt sich auch bis zum Oberrand des Beutels aus, also in einen Bereich hinein, der teilweise von dem reflektierenden Material abgeschirmt ist. Aus diesem Grunde wird sich hier eine Temperaturdifferenz nicht aufbauen können. Lediglich das geringe Restvolumen, welches unmittelbar in den Stutzen bzw. schlauchförmigen Verlängerungen des Beutels eingeschlossen ist, erreicht während des Erwärmungsvorgangs nicht die Endtemperatur. Dieses geringe Restvolumen ist aber vernachlässigbar. Die Bildung von Koageln im Blut bzw. Gerinnungsrückstanden am Blutplasma wird jedenfalls mit Sicherheit vermieden, wobei der wesentliche Inhalt des Beutels auch rasch und gleichmäßig erwärmt wird. Da sich die Lage der Stutzen und schlauchförmigen Verlängerungen am Beutel nicht immer genau vorhersehen läßt, weil die Beutel auch in durchaus unterschiedlicher Weise hergestellt werden, muß der Bereich der Abschirmung entsprechend groß gewählt werden, was aber infolge der Schräg-Taumeldurchmischung nicht weiter nachteiligt ist.

Die Aufspannplatte und die Abdeckplatte weisen zweckmäßig auf ihren einander zugekehrten Seiten das reflektierende Material auf, welches in der Regel ein Metall, eine Metallfolie oder Metallplatte ist. Beim Auflegen des Beutels auf die Aufspannplatte bildet somit das dort sichtbare reflektierende Material eine Fläche, die es gestattet, den Beutel so relativ zu der Aufspannplatte zu plazieren, daß die gefährdeten Stutzen und Schlauchabschnitte innerhalb dieser Fläche zu liegen kommen. Von der anderen Seite wird dann der Beutel mit gleicher Fläche abschirmend durch die Abdeckplatte geschützt.

Die Aufspannplatte und die Abdeckplatte bestehen in aller Regel aus Kunststoff, weil dieser für Mikrowellen weitgehend durchlässig ist. Die beiden Platten können entsprechend der Anordnung von Stutzen am Beutel mit je einer Messingplatte versehen sein.

Die Messingplatten können auf der der Drehachse des Elektromotors abgewandten Seite der Aufspannplatte und der Abdeckplatte angeordnet sein und sich etwa über ein Drittel der Länge der Aufspannplatte und der Abdeckplatte erstrecken. Dies ist für eine leichte und sichere Bedienung förderlich. Der Elektromotor des Drehantriebs hält zweckmäßig immer in derselben Relativlage der Aufspannplatte zur Horizontalen an, in der das Aufspannen bzw. Abspannen des Beutels erfolgt.

Für die Anordnung der Abdeckplatte relativ zur Aufspannplatte, also ihre gelenkige Verbindung aneinander und ihre Abschwenk- bzw. Fixierbewegung können in ganz verschiedener Weise realisiert werden. Wesentlich ist eigentlich nur, daß die Abdeckplatte in der Spannstellung etwa parallel zu der Aufspannplatte ausgerichtet fixiert wird, und daß ein gewisser Druck zwischen den Platten entsteht, zwischen denen der Beutel flachgepreßt gehalten wird. Da es Beutel unterschiedlicher Größe gibt, ist es zweckmäßig, daß die Abdeckplatte eine Durchbrechung aufweisen kann, durch die ein Spanngurt verschieblich hindurchgeführt ist, dessen freies Ende mit der Aufspannplatte lösbar verbunden ist. Damit kann eine gewisse Ausrichtung der Abdeckplatte in ihrer Parallelität zur Aufspannplatte erreicht werden, die auch bei unterschiedlichen Beutelinhalten wirksam wird bzw. eine entsprechende Ausrichtung erlaubt.

Die Erfindung wird anhand bevorzugter Ausführungsbeispiele weiter beschrieben. Es zeigen :

Figur 1 eine schematische Darstellung des Mikrowellengeräts in einer ersten Ausführung,

Figur 2 eine perspektivische Darstellung der für die Erfindung wesentlichen Teile der Haltevorrichtung des Mikrowellengeräts bei einer zweiten Ausführung,

Figur 3 eine Draufsicht auf die Teile gemäß Figur 2 von oben und

Figur 4 eine Seitenansicht der Teile gemäß Figur 2.

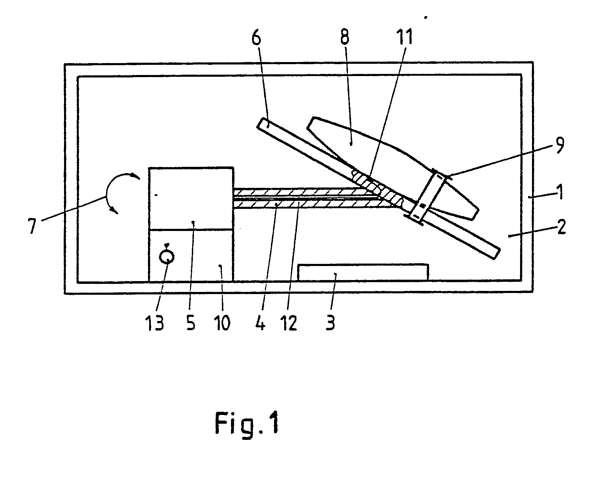

In einem in Figur 1 nur angedeuteten Gehäuse (1), welches mit einer Tür oder einen anderen Verschlußeinrichtung ausgestattet ist, ist ein Innenraum (2) gebildet, in welchem ein Erzeuger (3) für Mikrowellen angebracht ist, etwa wie in einem Mikrowellenherd. In dem Innenraum (2) ist außerdem eine Welle (4) drehbar gelagert, die von einem Elektromotor (5) hin- und herschwenkend angetrieben wird. Am freien Ende der Welle (4) ist eine Aufspannplatte (6) schrägstehend angeordnet, die mit der Horizontalen in ihrer ausgerichteten Mittelstellung etwa einen Winkel von 45° einschließt. Über den Elektromotor (5) ist somit die Aufspannplatte (6) in einem Verschwenkwinkel von etwa 350° gemäß Pfeil (7) hin- und herschwenkbar angeordnet. Die Aufspannplatte (6) besteht aus Kunststoff, der sich nicht oder möglichst wenig infolge der Mikrowellen des Erzeugers (3) erwärmt. Die Aufspannplatte (6) dient der Aufnahme eines Beutels (8) des Blutes oder des Blutderivates, welches erwärmt werden soll. Vermitels einer an der Aufspannplatte (6) angeordneten Spanneinrichtung, Spannbügel o. dgl. (9) wird der Beutel (8) an der Aufspannplatte (6) fixiert. Außerdem ist im Innenraum (2) eine Steuereinrichtung (10) angeordnet. Über ein Thermoelement (11) in der dem Beutel (8) zugekehrten Oberfläche der Aufspannplatte (6) wird die Temperatur des Beutels (8) bzw. des darin befindlichen Blutes gemessen. Von dem Thermoelement (11) führt eine Leitung (12) zu der Steuereinrichtung (10), die den Erzeuger (3) der Mikrowellen steuert. Über einen Verstellknopf (13) kann die gewünschte Endtemperatur des Bluts nach der Aufheizung festgelegt werden. Diese beträgt normalerweise 27°, kann aber auch auf andere Werte, z. B. 33 °C eingestellt werden. Die Aufheizzeit einer Blutkonserve in einem Beutel mit 400 bis 500 ml Inhalt von 4 °C auf 32 °C beträgt ca. 110 sec. Durch den hin- und hergehenden reversierenden Antrieb der Aufspannplatte (6) über den Motor (5) wird der Beutel (8) während der Erwärmung in eine Schraub-Dreh-Bewegung versetzt, durch die eine besonders gute Durchmischung des Bluts stattfindet, so daß das gesamte in dem Beutel (8) befindliche Blut gleichmäßig erwärmt wird.

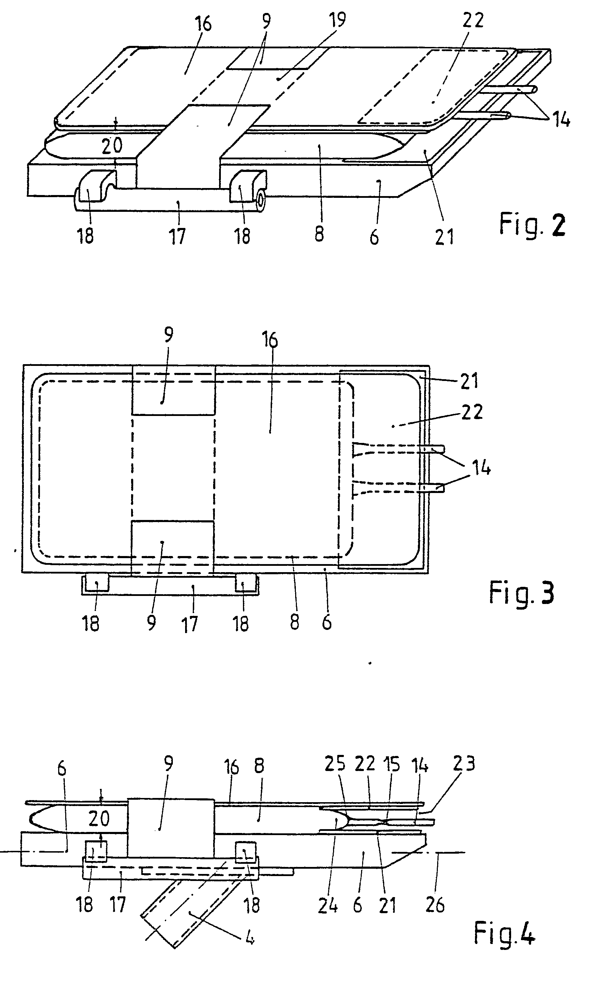

Der Beutel (8), der gewöhnlich aus Kunststoffolie besteht, nimmt die zu erwärmende Flüssigkeit, nämlich das Blut bzw. die Blutderivate auf. Der Beutel (8) besitzt etwa rechteckige, flache Form und trägt entlang seines oberen Randes meist eine Mehrzahl von Stutzen (14) oder anderen schlauchförmigen Verländerungen, die als Einfüll- bzw. Entnahmeöffnungen genutzt werden oder anderen Zwecken dienen. In Figur 4 ist der Abschluß (15) an den Stutzen (14) dargestellt, so daß die Flüssigkeit im Beutel (8) bis zu diesem Abschluß (15) ansteht.

Die Haltevorrichtung für den Beutel weist eine Aufspannplatte (6) auf, die also eine ebene Oberfläche besitzt, auf der sich der Beutel (8) auflegt. Parallel zu der Aufspannplatte, jedoch im Abstand dazu, ist eine Abdeckplatte (16) vorgesehen, die ähnliche Flächenausdehnung wie die Aufspannplatte (6) aufweist und natürlich ebenfalls als flache Fläche ausgebildet ist. Ein Spanngurt (9) ist mit einem Ende an der Aufspannplatte (6) befestigt und kann mit seinem anderen Ende mit Hilfe eines dort angeordneten Knebels (17) in zwei Haken (18) lösbar eingehängt werden. Der Spanngurt (4) durchsetzt eine Durchbrechung (19) an der Abdeckplatte (16). Der Spanngurt (9) besteht aus elastisch-nachgiebigem Material, so daß die Abdeckplatte (16) relativ zum Spanngurt (9) so ausgerichtet und in der Spannstellung fixiert werden kann, daß sich die Abdeckplatte (16) möglichst genau parallel und im Abstand zur Aufspannplatte (6) befindet. Der Beutel (8) wird somit flachgepreßt und über seine wesentliche Flächenerstreckung mit konstanter Schichtdicke (20) in der Haltevorrichtung gehalten.

Im oberen Bereich des Beutels (8), dort, wo die Stutzen (14) vorgesehen sind bzw. sich der Oberrand des Beutels befindet, sind die Aufspannplatte (6) und die Abdeckplatte (16) auf den einander zugekehrten Flächen mit zwei Messingplatten (21 und 22) versehen oder in anderer Weise mit einem die Mikrowellen reflektierenden Material belegt. Die Anordnung der Messingplatten (21 und 22), d. h. ihre flächenmäßige Erstreckung und ihre Plazierung innerhalb der Aufspannplatte (6) und der Abdeckplatte (16) sind so gewählt, daß sich der Oberrand des Beutels (8) einschließlich des Teils der Stutzen, der bis zum Abschluß (15) führt, auf jeden Fall innerhalb des Umrisses der Messingplatten (21 und 22) befinden. Diese Messingplatten (21 und 22) schirmen die im Feld zwischen ihnen angeordneten Teile des Beutels bzw. der Stutzen (14) vor der übermäßigen Einwirkung von Mikrowellen ab. Selbstverständlich können durch den zwischen den Messingplatten (21 und 22) gebildeten Spal (23) seitlich Mikrowellen eindringen, die in erwünschter Weise zu einer vergleichsweise langsameren und damit in der Temperatur zurückbleibenden Erwärmung der Flüssigkeit im Stutzen (14) bis zum Abschluß (15) führen. Die Flüssigkeit an der Stelle (24), also etwa entlang des Oberrandes (25) des Beutels (8), wird zwar auch von den Messingplatten (21 und 22) teilweise abgeschirmt. Diese Stelle (24) nimmt jedoch an der besonderen Schräg-Taumeldurchmischung des Hauptinhalts des Beutels (8) teil, so daß sich hier Temperaturdifferenzen zu dem übrigen Beutelinhalt nicht aufbauen können.

Figur 4 zeigt auch die Relativlage zwischen der Drehwelle (4) und der Achse (26) der Aufspannplatte (6) bzw. der gesamten Haltevorrichtung. Diese Achsen (4 und 26) sind schrägstehend zueinander, also in einem von 90° verschiedenen Winkel angeordnet. Die Verlängerung der Welle (4) bzw. des entsprechenden Wellenstummels führt bzw. entspricht der Achse des Elektromotors für den Drehantrieb. Die Welle (4) kann vorzugsweise horizontal ausgerichtet angeordnet sein, so daß an dem Mikrowellengerät die Achse (26) letztlich schräg zur Horizontalen angeordnet ist, also in einem von 90° verschiedenen Winkel zur Horizontalen.

## Patentansprüche

1. Mikrowellengerät zum Erwärmen von in einem Beutel bevorratetem Blut oder Blutderivaten, mit einem Mikrowellenerzeuger und einer Aufnahmevorrichtung mit im wesentlichen ebener Fläche für den Beutel, deren Drehachse quer zu der Drehachse eines reversierend angesteuerten Antriebes angeordnet ist, dadurch gekennzeichnet, daß die Aufnahmevorrichtung in einem Mikrowellenerwärmungsraum vorgesehen ist und eine Aufspannplatte (6) zur Aufnahme des Beutels (8) aufweist, daß die ebene Fläche der Aufspannplatte (6) schrägstehend zu der Drehachse (4) des als Elektromotors (5) ausgebildeten Antriebs angeordnet und mit einem Ende der Drehachse (4) verbunden ist.

2. Mikrowellengerät nach Anspruch 1, dadurch gekennzeichnet, daß die Aufspannplatte (6) etwa in einem Winkel von 45° zur Horizontalen angeordnet ist.

3. Mikrowellengerät nach Anspruch 1 und 2, dadurch gekennzeichnet, daß die Aufnahmevorrichtung einen Fixiergurt, einen Spannbügel (9) o. dgl. zur Fixierung des Beutels (8) auf der Aufspannplatte (6) aufweist.

4. Mikrowellengerät nach Anspruch 1 oder 2, dadurch gekennzeichnet, daß in der dem Beutel zugekehrten Oberfläche der Aufspannplatte (6) ein Thermoelement (11) zur Steuerung des Mikrowellenerzeugers (3) vorgesehen ist.

5. Mikrowellengerät nach Anspruch 1 und 2, dadurch gekennzeichnet, daß die Aufnahmevorrichtung eine Abdeckplatte (16) aufweist, zwischen der und der Aufspannplatte (6) der Beutel (8) im Sinne gleicher Schichtdicken (20) flachgepreßt gehalten ist, und daß die Aufspannplatte (6) und die Abdeckplatte (16) über einen Teil ihrer Längs- und Querausdehnung entsprechend der Anordnung der Stutzen (14) am Beutel (8) mit einem die Mikrowellen reflektierenden Material belegt sind.

6. Mikrowellengerät nach Anspruch 5, dadurch gekennzeichnet, daß die Aufspannplatte (6) und die Abdeckplatte (16) auf ihren einander zugekehrten Seiten das reflektierende Material aufweisen.

7. Mikrowellengerät nach Anspruch 5 oder 6, dadurch gekennzeichnet, daß die Aufspannplatte (6) und die Abdeckplatte (16) aus Kunststoff bestehen und entsprechend der Anordnung von Stutzen (14) am Beutel (8) mit je einer Messingplatte (21, 22) versehen sind.

8. Mikrowellengerät nach Anspruch 7, dadurch gekennzeichnet, daß die Messingplatten (21, 22) auf der der Drehachse (4) des Elektromotors abgewandten Seite der Aufspannplatte (6) und der Abdeckplatte (16) angeordnet sind und sich etwa über ein Drittel der Länge der Aufspannplatte (6) und der Abdeckplatte (16) erstrecken.

9. Mikrowellengerät nach Anspruch 5 bis 8, dadurch gekennzeichnet, daß die Abdeckplatte (16) eine Durchbrechung (19) aufweist, durch die ein Spanngurt (9) verschieblich hindurchgeführt ist, dessen freies Ende mit der Aufspannplatte (6) lösbar verbunden ist.

## Claims

1. Microwave appliance, for the heating of blood or blood derivatives stored in a sachet, with a microwave generator and a receiving device of substantially planar area for the sachet, the rotational axis of which area is arranged obliquely to the rotational axle of a reversingly driven drive, characterised thereby, that the receiving device is provided in a microwave heating space and displays a clamping plate (6) for the reception of the sachet (8), that the planar area of the clamping plate (6) is arranged obliquely to the rotational axle (4) of the drive constructed as electrical motor (5) and connected with one end of the rotational axle (4).

2. Microwave appliance according to claim 1, characterised thereby, that the clamping plate (6) is arranged at about an angle of 45° to the horizontal.

3. Microwave appliance according to claim 1 and 2, characterised thereby, that the receiving device displays a fixing belt, a clamping clip (9) or the like for the fixing of the sachet (8) on the clamping plate (6).

4. Microwave appliance according to claim 1 or 2, characterised thereby, that a thermocouple element (11) for the control of the microwave generator (3) is provided in that surface of the clamping plate (6), which faces the sachet.

5. Microwave appliance according to claim 1 and 2, characterised thereby, that the receiving device displays a cover plate (16), between which and the clamping plate (6), the sachet (8) is held pressed flat in the sens of equal layer thicknesses and that the clamping plate (6) and the cover plate (16) are coated with a material reflecting the microwaves over a part of their longitudinal and transverse extent in correspondence with the arrangement of the nipples (14) at the sachet (8).

6. Microwave appliance according to claim 5, characterised thereby, that the clamping plate (6) and the cover plate (16) display the reflecting material on their mutually facing sides.

7. Microwave appliance according to claim 5 or 6, characterised thereby, that the clamping plate (6) and the cover plate (16) consist of synthetic material and are each provided with a respective brass plate (21, 22) in correspondence with the arrangement of the nipples (14) at the sachet (8).

8. Microwave appliance according to claim 7, characterised thereby, that the brass plates (21, 22) are arranged on that side of the clamping plate (6) and the cover plate (16), which is remote from the rotational axle (4) of the electrical motor, and extend over about one third of the length of the clamping plate (6) and the cover plate (16).

9. Microwave appliance according to claim 5 to 8, characterised thereby, that the cover plate (16) displays a passage (19), through which a tightening belt (9) is displaceably led, the free end of which is detachably connected with the clamping plate (6).

## Revendications

1. Dispositif à micro-ondes pour réchauffer du sang ou des dérivés du sang contenus dans une poche, avec un générateur de micro-ondes et un dispositif de support ayant une surface essentiellement plane pour recevoir la poche, l'axe de rotation de ce dispositif étant disposé transversalement à l'axe de rotation d'un moyen d'entraînement commandé de manière réversible, caractérisé en ce que le dispositif de support est prévu dans une chambre de chauffage par micro-ondes et présente une plaque de support (6) pour recevoir la poche (8), en ce que la surface plane de la plaque de support (6) est disposée obliquement par rapport à l'axe de rotation (4) du moyen d'entraînement constitué par un moteur électrique (5) et est fixée sur une extrémité de l'axe de rotation (4).

2. Dispositif à micro-ondes selon la revendication 1, caractérisé en ce que la plaque de support (6) est disposée selon un angle d'à peu près 45° par rapport à l'horizontale.

3. Dispositif à micro-ondes selon les revendications 1 et 2, caractérisé en ce que le dispositif récepteur présente une sangle (9), un étrier de serrage ou un élément similaire pour fixer la poche (8) sur la plaque de support.

4. Dispositif à micro-ondes selon la revendication 1 ou 2, caractérisé en ce qu'une sonde thermo-électrique (11) est prévue dans la face de la plaque de support (6) tournée vers la poche pour commander le générateur de micro-ondes (3).

5. Dispositif à micro-ondes selon les revendications 1 et 2, caractérisé en ce que le dispositif récepteur présente une plaque de recouvrement (16), la poche (8) étant maintenue entre cette plaque (16) et la plaque de support (6) en étant serrée à plat de manière à présenter une épaisseur (20) uniforme, et en ce que la plaque de support (6) et la plaque de recouvrement (16) sont garnies sur une partie de leur longueur et de leur largeur d'un matériau réfléchissant les micro-ondes, en fonction de la disposition des tubulures (14) sur la poche (8).

6. Dispositif à micro-ondes selon la revendication 5, caractérisé en ce que la plaque de support (6) et la plaque de recouvrement (16) présentent le matériau réfléchissant sur leurs faces se faisant vis-à-vis.

7. Dispositif à micro-ondes selon la revendication 5 ou 6, caractérisé en ce que la plaque de support (6) et la plaque de recouvrement (16) sont en matière plastique et munies chacune d'une plaque de laiton (21, 22) en fonction de la disposition de tubulures (14) sur la poche (8).

8. Dispositif à micro-ondes selon la revendication 7, caractérisé en ce que les plaques de laiton (21, 22) sont disposées sur la face de la plaque de support (6) et de la plaque recouvrante (16) éloignée de l'axe de rotation (4) du moteur électrique et s'étendent sur environ un tiers de la longueur de la plaque de support (6) et de la plaque de recouvrement (16).

9. Dispositif à micro-ondes selon les revendications 5 à 8, caractérisé en ce que la plaque de recouvrement (16) présente une encoche (19) dans laquelle on fait passer de manière coulissante une sangle de serrage (9) dont l'extrémité libre est attachée de manière amovible sur la plaque de support (6).

Fig.1

Fig. 2

Fig. 3

Fig. 4